# EUROPÄISCHE PATENTSCHRIFT

(11) **EP 0 999 978 B1**
(45) Veröffentlichungstag und Bekanntmachung des Hinweises auf die Patenterteilung: **08.05.2002**
(21) Anmeldenummer: 98941359.6
(22) Anmeldetag: 18.07.1998
(51) Int. Cl.: B64D 45/00, G08B 13/196

(54) **ÜBERWACHUNGSEINRICHTUNG FÜR INNENRÄUME VON FLUGZEUGEN, INSBESONDERE PASSAGIERFLUGZEUGEN**
SURVEILLANCE DEVICE FOR USE IN AIRCRAFT INTERIORS, ESPECIALLY IN PASSENGER AEROPLANES
DISPOSITIF DE SURVEILLANCE POUR LES CABINES D'AERONEFS, NOTAMMENT D'AVIONS DE TRANSPORT DE PASSAGERS

(30) Priorität: 30.07.1997 DE 19732806
(43) Veröffentlichungstag der Anmeldung: 17.05.2000
(73) Patentinhaber: VIDAIR Aktiengesellschaft, 41238 Mönchengladbach (DE)
(72) Erfinder: RÖMMEN, Markus, D-41238 Mönchengladbach (DE)
(74) Vertreter: Stenger, Watzke & Ring Patentanwälte
(86) Internationale Anmeldenummer: EP9804473
(87) Internationale Veröffentlichungsnummer: WO9906275

(56) Entgegenhaltungen:
- EP-A- 0 232 031
- BE-A- 759 331
- CH-A- 651 984
- FR-A- 2 753 588
- US-A- 4 831 438
- US-A- 5 144 661
- PATENT ABSTRACTS OF JAPAN vol. 097, no. 005, 30. Mai 1997 & JP 09 020297 A (NIPPON SEIGYO:KK), 21. Januar 1997

## Beschreibung

Die Erfindung betrifft eine Überwachungseinrichtung für Innenräume von Flugzeugen, insbesondere Passagierflugzeugen.

Unerwartete Vorfälle an Bord von Flugzeugen sorgen immer wieder für schlagzeilenträchtige Ereignisse. Hierunter fallen Störungen des Flugbetriebes infolge menschlichen oder technischen Versagens bis hin zu Flugzeugabstürzen, aber auch Ereignisse wie Flugzeugentführungen oder andere Krisensituationen in der Passagierkabine oder dem Cockpit des Flugzeuges.

Dabei stellt sich immer wieder als problematisch heraus, daß Informationen über das Geschehen an Bord letztlich nur durch mündlichen Bericht, zumeist durch den Piloten oder Copiloten, an das Bodenpersonal weitergegeben und für dort zu treffende Entscheidungen ausgewertet werden können. Auch die heute in allen Flugzeugen der Zivilluftfahrt vorgeschriebene "Blackbox", in der technische Flugdaten sowie die Gespräche zwischen Cockpit und nächstliegendem Flughafentower aufgezeichnet werden, kann über viele Geschehnisse an Bord nicht oder nur unvollständig Auskunft geben.

Aus der US 5,144,661 ist ein Sicherheitssystem bekannt, welches dazu dient Video- und Audiosignale aufzunehmen, diese zu speichern und anschließend an eine Gegenstation zur Verarbeitung zu übermitteln. Hierbei besteht das Sicherheitssystem im wesentlichen aus Störungen erkennenden Sensoren, einer Video- und Aufzeichnungseinheit sowie einer Kontrolleinheit zur Steuerung der Video- und Aufzeichnungseinheit.

Sobald die Sensoren eine Störung detektieren wird die Kontrolleinheit aktiviert, die ihrerseits die Aufzeichnungseinheit ansteuert. Die Video- und Audiosignale werden sodann in ein digitales Format konvertiert und auf einer magnetischen Festplatte gespeichert. Die konvertierten Signale werden ferner für eine Überwachung der von den Sensoren erfaßten Ereignisse zu einer Basisstation übermittelt. Hierbei ist neben einer Aktivierung der Kontrolleinheit durch die Sensoren auch eine manuelle sowie eine durch die Bodenstation veranlaßte Aktivierung der Kontrolleinheit möglich.

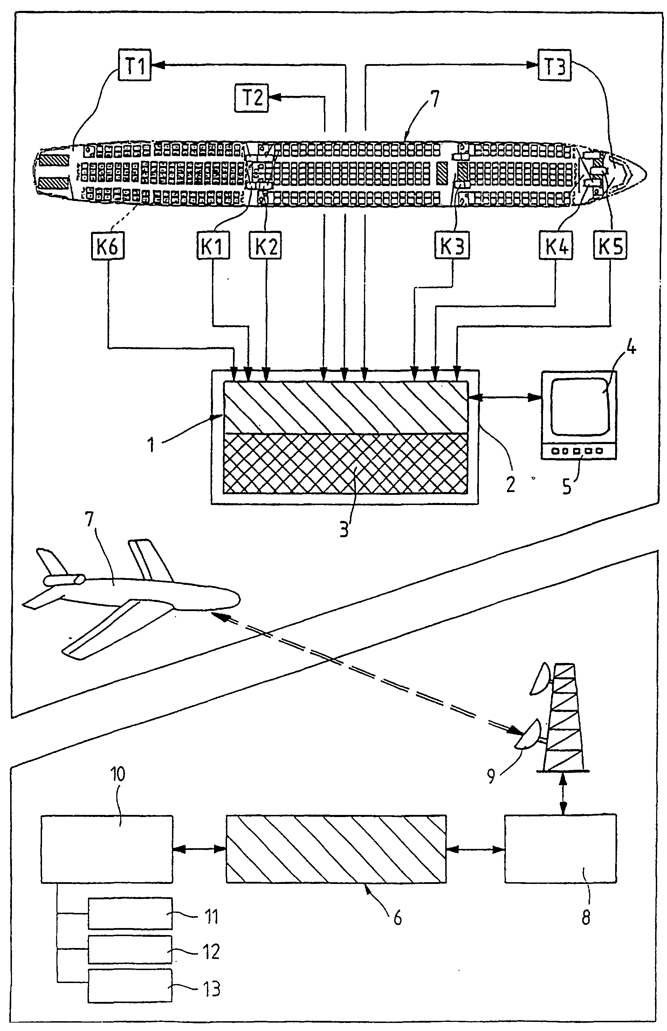

Gemäß Fig. 4 dieser Entgegenhaltung ist dieses Sicherheitssystem für die Verwendung in enem Flugzeug offenbart. Es kann unabhängig von anderen, bereits an Bord des Flugzeugs installierten Sicherheitssystemen betrieben werden und soll die Geschehnisse im Innenraum des Flugzeugs, insbesondere im Fall einer Flugzeugentführung, aufzeichnen und entsprechende Signale an eine Bodenstation senden. Zu diesem Zweck sind in den Toiletten, der Passagierkabine sowie dem Cockpit Störungen erkennende Sensoren sowie Aufzeichnungseinheiten in einer den gesamten Innenraum des Flugzeugs überwachenden Anzahl vorhanden. Als Aufzeichnungseinheiten kommen dabei sowohl Video- als auch Audioeinheiten in Frage. Die von den Aufzeichnungseinheiten gelieferten Signale werden gespeichert und/oder mittels einer Flugzeug-Bodenstation-Kommunikationseinheit an die jeweils nächste Bodenstation übermittelt, wo die empfangenen Daten mittels einer Rechneranlage ausgewertet werden können. Sollte ein Übertragen der Daten an die Bodenstation nicht möglich sein, so werden die Video- und Audiodaten gespeichert und nach einer Landung des Flugzeugs ausgelesen. Gemäß einer bevorzugten Ausführungsform steht das an Bord des Flugzeugs integrierte Sicherheitssystem mit der Bodenstation in kommunikativer Verbindung.

Die EP 0 232 031 offenbart ein Überwachungssystem für Flugzeuge mit wenigstens einer in einem Flugzeug angeordneten Überwachungskamera für die Überwachung eines vorgebbaren Bereichs. Desweiteren ist ein Signalaufnehmer im Flugzeug vorgesehen, der die Videosignale der Kamera in Audiosignale umwandelt, die dann anschließend von einer ebenfalls im Flugzeug angeordneten Sender-/Empfängereinheit an eine am Boden befindliche Kommandostation übermittelt wird. Für den Empfang der von der flugzeugeigenen Sende-/Empfängereinheit abgegebenen Signale verfügt die Kommandostation gleichfalls über eine entsprechende Sende/Empfängereinheit. Die Kommandostation verfügt desweiteren über einen mit einem Monitor verbundenen Signalaufnehmer, der die empfangenen Audiosignale in Videosignale umwandelt. Zur Steuerung der im Flugzeug angeordneten Kamera ist eine im Flugzeug befindliche Kontrolleinheit vorgesehen, die sowohl flugzeugintern als auch über die Kommandostation ansteuerbar ist.

Ausgehend von diesem Stand der Technik liegt der Erfindung die Aufgabe zugrunde, die Sicherheit in Flugzeugen und insbesondere in Passagierflugzeugen im Falle unerwarteter Ereignisse zu erhöhen.

Hierzu wird eine Überwachungseinrichtung für Innenräume von Flugzeugen, insbesondere Passagierflugzeugen vorgeschlagen, mit
- an Bord des Flugzeuges installierten Bilderfassungsgeräten, wobei wenigstens ein Bilderfassungsgerät den Cockpitbereich einschließlich des Tätigkeitsbereichs der Piloten abdeckt,
- einer bordeigenen Kommunikationseinheit mit Eingängen für die Bilderfassungsgeräte sowie einer Speichereinheit zur vorzugsweise komprimierten Abspeicherung der von den Bilderfassungsgeräten gelieferten Bilddaten,
- einer mit der bordeigenen Kommunikationseinheit in Datenaustausch stehenden stationären Kommunikationseinheit mit daran angeschlossenen Einrichtungen zur Bilddatenverarbeitung und -wiedergabe,
- Sende- und Empfangseinrichtungen zur Übertragung zumindest der Bilddaten von der bordeigenen an die stationäre Kommunikationseinheit und Steuerdaten von der stationären an die bordeigene Kommunikationseinheit,
wobei die stationäre Kommunikationseinheit mit Mitteln zur Isolierung der Bilddaten und gegebenenfalls Steuerdaten aus der allgemeinen Funkleitstrahlinformation zwischen Flugzeug und Bodenstation versehen ist.

Mit einer solcherart ausgestalteten Überwachungseinrichtung wird die Möglichkeit geschaffen, im Falle unerwarteter Ereignisse in einem Flugzeug Bilddokumente aus der Zeit vor und während des Ereignisses durch Fachleute am Boden auszuwerten. Die Sicherheit für die Fluggäste wird erhöht, ferner wird ein Beitrag dazu geleistet, im Falle einer gravierenden Störung bis hin zur Katastrophe die Frage nach menschlichem oder technischem Versagen besser zu beantworten. Die Überwachungseinrichtung eignet sich grundsätzlich für jeden Flugzeugtyp, da in der internationalen Luftfahrt die Kommunikationswege zwischen den Flugzeugen und den jeweiligen bodengestützten Einrichtungen weitgehend genormt sind. Besondere Bedeutung erlangt die Überwachungseinrichtung bei einer Flugzeugentführung. In diesem Fall lassen sich mittels der ausgewerteten Bilddaten durch Fachleute am Boden Entscheidungen über Gegenmaßnahmen treffen, und gegebenenfalls entsprechende Vorbereitungen bereits vor der Landung des Flugzeuges treffen.

Wenn die Bilddaten und die Steuerdaten für die bordeigene Kommunikationseinheit gemeinsam mit den zwischen Flugzeug und Tower ausgetauschten Flugdaten übermittelt werden, ermöglicht die mit Mitteln zur Isolierung der Bilddaten und gegebenenfalls Steuerdaten aus der allgemeinen Kommunikationsverbindung zwischen Flugzeug und Flughafentower versehene stationäre Kommunikationseinheit eine Herausfilterung dieser speziellen Daten aus der allgemeinen Kommunikationsverbindung, in der Regel der Funkleitstrahlinformation. Dadurch ist ein die Sicherheit erhöhendes schnelleres Auswerten der Daten ermöglicht.

Gemäß einer bevorzugten Weiterbildung der Überwachungseinrichtung wird vorgeschlagen, daß die stationäre Kommunikationseinheit Mittel zur Erzeugung von Steuerdaten für den Betrieb der bordeigenen Kommunikationseinheit aufweist. Auf diese Weise läßt sich etwa vom Tower des nächstgelegenen Flughafens aus die an Bord befindliche Kommunikationseinheit ansteuern, um gewünschte Bilddaten vom Flugzeug abzurufen. Diese können dann von dem Bodenpersonal ausgewertet werden. Auch kann der an Bord befindlichen Kommunikationseinheit mittels der Steuerdaten signalisiert werden, bestimmte Bilddaten, beispielsweise jene einer bestimmten Kamera, zu übertragen. Auch können über die Steuerdaten einzelne Funktionen der bordeigenen Kameras, zum Beispiel die Zoomfunktion oder ein Verschwenken der Kameraachse, angesteuert werden.

Vorzugsweise ist zur Steuerung der bordeigenen Kommunikationseinheit und zur Bilddatenverarbeitung und -wiedergabe ein Personal Computer mit Monitor an die stationäre Kommunikationseinheit angeschlossen.

Mit einer weiteren Ausgestaltung wird vorgeschlagen, daß als Sende- und Empfangseinrichtung des Flugzeugs die vorhandene Bordantenne dient. Demgegenüber können als stationäre Sende- und Empfangseinrichtungen die land- oder satellitengestützten Einrichtungen der weltweit verbreiteten Bodenstationen dienen, d.h. vor allem die Flughafentower.

Um auch im Falle eines schweren Unfalles oder sogar Absturzes auf die gespeicherten Bilddaten zurückgreifen zu können, wird ferner vorgeschlagen, daß die Speichereinheit mit einer spannungsversorgungsunabhängigen Datensicherung versehen ist, wie zum Beispiel einer magnetischen Festplatte oder einem anderen nichtflüchtigen Speichermedium, und daß sich die bordeigene Kommunikationseinheit einschließlich der Speichereinheit in einem geschlossenen Gehäuse befindet, welches wasserdicht und stoßgesichert ist.

Mit der Erfindung wird ferner mindestens ein im Innenraum des Flugzeuges angeordneter Alarmtaster sowie eine Steuerung in der bordeigenen Kommunikationseinheit, die durch Betätigung des Alarmtasters selbsttätig eine Übertragung von Signalen und vorzugsweise Bilddaten an die stationäre Kommunikationseinheit auslöst, vorgeschlagen. Erfahrungsgemäß steht dem Bordpersonal im Ernstfall nicht die Zeit zur Verfügung, sich mit der nächstgelegenen Bodenstation in Verbindung zu setzen, um von dort die erforderlichen Bildinformationen abrufen zu lassen. Es ist daher von großem Vorteil, wenn alleine durch Betätigung des Alarmtasters ein Automatismus ausgelöst wird, bei dem die erforderlichen Signale und vor allem Bilddaten selbsttätig an die stationäre Kommunikationseinheit übertragen werden.

Zu den besonders kritischen Situationen an Bord eines Passagierflugzeuges zählen Flugzeugentführungen. Hierbei verschaffen sich die Entführer zunächst Zutritt zum Cockpit des Flugzeuges, und dies notfalls auch mit Gewalt. Sicherungseinrichtungen zwischen Passagierkabine und Cockpit sind daher auch weitgehend wirkungslos, da erfahrungsgemäß das Bordpersonal mit Waffengewalt gezwungen wird, diese freizugeben. Es ergibt sich gemäß einer Weiterbildung der Erfindung jedoch die Möglichkeit, daß im Innenraum mindestens eine an die bordeigene Kommunikationseinheit angeschlossene Eingabevorrichtung für einen Code, vorzugsweise einen Zahlencode angeordnet ist, daß die bordeigene Kommunikationseinheit mit einem Vergleichsoperator zum Vergleich des eingegebenen Codes mit einem gespeicherten Nötigungscode versehen ist, und daß die bordeigene Kommunikationseinheit mit einer im Falle einer Übereinstimmung zwischen eingegebenem Code und gespeichertem Nötigungscode ausgelösten Steuerung versehen ist, die selbsttätig eine Übertragung von Signalen und vorzugsweise Bilddaten an die stationäre Kommunikationseinheit auslöst. Das Bordpersonal arbeitet also mit zwei unterschiedlichen Codes, einem Normalcode für alle üblichen Situationen, in denen das gesicherte Cockpit betreten werden muß, und einem Dritten nicht bekannten Nötigungscode. Bei dessen Eingabe erfolgt zusätzlich zu der Öffnungsfunktion und für den Dritten nicht wahrnehmbar die Übertragung der Signale und Bilddaten an die stationäre Kommunikationseinheit.

Um auch der Cockpitbesatzung eine Abfrage der einzelnen Bilddaten zu ermöglichen, wird ein an die bordeigene Kommunikationseinheit angeschlossener Monitor zur wahlweisen Wiedergabe der von den Bilderfassungsgeräten gelieferten Bilddaten vorgeschlagen. Hierbei können die einzelnen Kameras getrennt angewählt und deren Bilddaten auf dem Monitor angezeigt werden. Alternativ ist es möglich, im Wege einer Zeitschaltung nacheinander die Bilder der einzelnen Kameras auf dem im Cockpit installierten Monitor anzuzeigen.

Weitere Vorteile und Einzelheiten der Überwachungseinrichtung ergeben sich aus der nachfolgenden Beschreibung eines auf der Zeichnung dargestellten Ausführungsbeispieles der Erfindung. Die Zeichnung zeigt in schematischer Darstellung eine Überwachungseinrichtung für Innenräume von Passagierflugzeugen unter Verwendung einer bordeigenen Kommunikationseinheit sowie einer stationären Kommunikationseinheit.

Die Zeichnung zeigt im oberen Teil die Innenaufteilung eines Passagierflugzeuges mit Passagierraum und Cockpit. Unterhalb des Passagierraumes befindet sich der Fracht- bzw. Laderaum. Bilderfassungsgeräte, vorzugsweise Videokameras, sind an verschiedenen Stellen innerhalb des Passagierflugzeuges angebracht.

Die Bilderfassungsgeräte K1, K2, K3 und K4 überwachen die einzelnen Zonen des Passagierraumes. Um eine unauffällige Unterbringung in dem Passagierraum zu erreichen, werden für die Bilderfassungsgeräte K1, K2, K3 und K4 Videokameras mit Nadelöhr-Objektiven eingesetzt.

Das Bilderfassungsgerät K5 deckt den Cockpitbereich und dort insbesondere den unmittelbaren Tätigkeitsbereich von Pilot und Copilot ab. Ein weiteres Bilderfassungsgerät K6 schließlich ist im Fracht-und Laderaum des Flugzeugs angeordnet und dient der Überwachung der dortigen Vorgänge.

Über Glasfaserkabel sind die Bilderfassungsgeräte K1 bis K6 an eine bordeigene Kommunikationseinheit 1 angeschlossen. Diese befindet sich an einer Stelle innerhalb des Flugzeuges, die für Unbefugte nicht ohne weiteres erreichbar ist. Ebenfalls über Glasfaserkabel sind mehrere Alarmtaster T1 bis T3 an die bordeigene Kommunikationseinheit 1 angeschlossen. Über die Alarmtaster T1 bis T3 können selbsttätige Vorgänge innerhalb der Kommunikationseinheit 1 ausgelöst werden.

Bestandteil der in einem geschlossenen, wasserdichten und stoßgesicherten Gehäuse 2 untergebrachten Kommunikationseinheit 1 ist eine Speichereinheit 3 für die von den Bilderfassungsgeräten K1 bis K6 gelieferten Bilddaten. Um diese Bilddaten und eventuell noch weitere Daten, wie zum Beispiel Steuerdaten, auch im Fall einer Unterbrechung der Spannungsversorgung aufrechtzuerhalten, ist die Speichereinheit 3 mit einer spannungsversorgungs unabhängigen Datensicherung versehen, zum Beispiel einer oder mehreren magnetischen Festplatten.

Die Kapazität der Speichereinheit 3 ist ausreichend, um sämtliche Bilddaten auch für den Zeitraum eines längeren Fluges, zum Beispiel eines Interkontinental-Fluges, vollständig abzuspeichern. Zur Einsparung von Speicherplatz wird ein Verfahren zur Bilddaten-Kompression mit einer Kompressionsrate größer als 1:100 eingesetzt.

Neben der Speichereinheit 3 besteht die bordeigene Kommunikationseinheit 1 auch aus einer Steuereinheit. Diese ist zum Beispiel in der Lage, einzelne der Bilderfassungsgeräte K1 bis K6 anzuwählen, Bilddaten an einen Monitor 4 zu übertragen, der im Cockpit im Blickbereich von Pilot und Copilot angeordnet ist sowie die einzelnen Bilderfassungsgeräte K1 bis K6 hinsichtlich ihrer Funktion zu beeinflussen, zum Beispiel durch einen Zoom oder ein Verschwenken der betreffenden Videokamera. Insbesondere sind die Funktionen der bordeigenen Kommunikationseinheit 1 von außerhalb beeinflußbar, wie nachfolgend noch näher beschrieben wird.

Der im Cockpit angeordnete Monitor 4 ist mit einem Bedienpanel 5 verbunden, über das sich einzelne Videokameras anwählen lassen. Alternativ kann an dem Bedienpanel 5 eine Schaltung angewählt werden, bei der die Kommunikationseinheit 1 abwechselnd die Bilddaten der einzelnen Videokameras auf den Monitor überträgt, so daß der Cockpitbesatzung eine laufende Überwachung der wichtigsten Bereiche des Flugzeug-Innenraums möglich ist. Der im Cockpit angeordnete Monitor 4 ist mit einer automatischen Dunkelschaltung versehen. In bestimmten Alarmfällen wird der Monitor 4 hierzu automatisch deaktiviert, um als Überwachungsinstrument z. B. nicht in die Hände eines Flugzeugentführers zu fallen.

Das im Cockpit montierte Bilderfassungsgerät K5 ist mit seinem Objektiv so ausgerichtet, daß eine Aufzeichnung aller Aktivitäten des Piloten und/oder des Copiloten möglich ist. Ferner erfolgt eine Aufzeichnung der Anzeige der vor dem Piloten angeordneten Bordinstrumente. Um selbst die Skalierung der Instrumente ausreichend klar darzustellen, handelt es sich bei dem Bilderfassungsgerät K5 um eine hochauflösende Videokamera, deren Bildsignale über ein Glasfaserkabel zu der bordeigenen Kommunikationseinheit 1 gelangen.

Das Bilderfassungsgerät K5 kann auch so angesteuert werden, daß der Kamerablick in Unendlichstellung auf das Cockpitfenster ausgerichtet ist. Hierdurch wird die Möglichkeit geschaffen, aufgrund der so übermittelten Bilddaten fernsteuerbare Landemanöver, etwa bei einem Ausfall sowohl des Piloten als auch des Copiloten, zu steuern.

Die bordeigene Kommunikationseinheit 1 steht im Datenaustausch mit einer stationären Kommunikationseinheit 6, die der Luftleitzentrale idealerweise jedes größeren internationalen Verkehrsflughafens zugeordnet und dort installiert ist.

Die drahtlose Signalverbindung zwischen der bordeigenen Kommunikationseinheit 1 und der jeweils nächstgelegenen stationären Kommunikationseinheit 6 erfolgt über vorhandene Sende- und Empfangseinrichtungen in der Luft und am Boden. Dies sind die Bordantenne des Flugzeuges 7 bzw. die an die nächstgelegene Bodenstation 8, zumeist ein Flughafentower, angeschlossene Bodenantenne 9 für die regulären Funkleitstrahlinformationen. Wenn auf der regulären Kommunikationsverbindung die zwischen den Kommunikationseinheiten 1, 6 ausgetauschten Daten und Signale herausgefiltert werden müssen, ist hierzu ist in den Kommunikationseinheiten 1, 6 jeweils eine entsprechende Software installiert.

Die zwischen der bordeigenen und der stationären Kommunikationseinheit ausgetauschten Daten werden in der stationären Kommunikationseinheit 6 insoweit aufgearbeitet, daß die Bearbeitung der anfallenden Daten in einem Personal Computer 10 erfolgen kann, an den wiederum ein Monitor 11, ein Modem 12, ein Faxgerät 13 oder noch weitere Peripherigeräte angeschlossen sein können. Das Videobild auf dem Monitor 11 gibt neben dem Bild selbst weitere Informationen wieder, und zwar die Nummer K1 bis K6 des jeweiligen Bilderfassungsgerätes, eine Identifikationsnummer für das Flugzeug, die Flugkoordinaten in Länge und Breite, die Geschwindigkeit des Flugzeuges, dessen Kurs über dem Grund sowie die Flughöhe.

Durch die Verbindung des Personal Computer 10 mit der stationären Kommunikationseinheit 6 ist es möglich, sämtliche Bilddaten der Speichereinheit 3 sowie alternativ in Echtzeit die aktuell von den Bilderfassungsgeräten K1 bis K6 gelieferten Bilddaten an den Boden zu übertragen und mittels des Personal Computers 10 zu bearbeiten, darzustellen, auszudrucken. Durch Steuerung der bordeigenen Kommunikationseinheit 1 mittels der in Daten- und Signalaustausch stehenden stationären Kommunikationseinheit 6 können ferner vom Personal Computer 10 aus Steuerungsvorgänge in der bordeigenen Kommunikationseinheit 1 ausgelöst werden. Hierzu erfolgt ein Austausch von Steuerdaten von der stationären auf die bordeigene Kommunikationseinheit. So können beispielsweise sämtliche für eines der Bilderfassungsgeräte K1 bis K6 bereits abgespeicherten Bilddaten abgerufen werden, um diese dann im Personal Computer 10 zu speichern und später abzurufen. Wegen der Bilddatenkompression erfolgt dies mit hoher Übertragungsgeschwindigkeit. Ferner können die aktuellen Bildsignale von jedem beliebigen der Bilderfassungsgeräte K1 bis K6 abgerufen und auf dem Monitor 11 angezeigt werden. Es eröffnet sich also die wichtige Möglichkeit, von außen, d.h. vom nächstgelegenen Flughafentower aus, in das Flugzeug "hineinzublicken", und zwar rückwirkend anhand der in der Speichereinheit 3 bereits abgespeicherten Bilddaten oder in Echtzeit und damit aktuell.

Von dieser Möglichkeit wird selbstverständlich nicht dauernd Gebrauch gemacht, sondern nur in den bereits genannten Gefahrensituationen. Die Übermittlung der Bilddaten an den Flughafentower kann in bestimmten Situationen selbsttätig erfolgen, ausgelöst durch ein bestimmtes technisches Ereignis, zum Beispiel eine besonders starke Erschütterung des Flugzeuges oder einen starken Druckabfall in der Kabine. Die Auslösung der Bilddatenübertragung kann mittels der Alarmtaster T1 bis T3 auch durch das Bordpersonal ausgelöst werden.

Die Art und der Umfang der selbsttätig übertragenen Daten hängt von der Natur des auslösenden Ereignisses ab. Im Extremfall, d.h. bei einem drohenden Absturz des Flugzeuges, wird die bordeigene Kommunikationseinheit 1 so viele Bilddaten wie möglich und in kürzester Zeit an die stationäre Kommunikationseinheit 6 übermitteln. Von besonderer Bedeutung ist hierbei die weitgehende Komprimierung der bereits angefallenen Bilddaten in der Speichereinheit 3, um so kürzeste Übertragungszeiten von der bordeigenen an die stationäre Kommunikationseinheit zu erzielen. Die Überwachungseinrichtung läßt sich auch als medizinische Datenbox zur Übermittlung medizinischer Daten von Bord an den Boden einsetzen. In medizinischen Notfällen an Bord kann am Ort des Geschehens ein weiteres, allerdings mobiles Bilderfassungsgerät K7 eingesetzt werden sowie ein ebenfalls mobiler Monitor. Unter Verwendung des Datenaustausches zwischen der bordeigenen Kommunikationseinheit 1 und der stationären Kommunikationseinheit 6 kann auf diese Weise eine Videokonferenz mit der Bodenstation geführt werden, so daß es einem Notarzt in der Bodenstation möglich ist, sich über die Situation an Bord visuell zu informieren und medizinische Hilfsmaßnahmen aufzuzeigen.

Femer ist ein Einsatz der Überwachungseinrichtung in Schulflugzeugen möglich. Zum Einsatz kommt hierbei die den Cockpitbereich erfassende Kamera K5, mit deren Hilfe die Bilddaten in Echtzeit an die Bodenstation übermittelt werden, wo der Fluglehrer diese Bilddaten erkennt und seinem im Cockpit befindlichen Flugschüler entsprechende Hinweise oder Anweisungen geben kann.

### Bezugszeichenliste

- 1: bordeigene Kommunikationseinheit
- 2: Gehäuse
- 3: Speichereinheit
- 4: Monitor
- 5: Bedienpanel
- 6: stationäre Kommunikationseinheit
- 7: Flugzeug
- 8: Bodenstation (Flughafentower)
- 9: Bodenantenne
- 10: Personal Computer
- 11: Monitor
- 12: Modem
- 13: Faxgerät

- K1 - K6: Videokameras (Bilderfassungsgeräte)
- T1 - T3: Alarmtaster

## Patentansprüche

1. Überwachungseinrichtung für Innenräume von Flugzeugen, insbesondere Passagierflugzeugen, mit
- an Bord des Flugzeuges installierten Bilderfassungsgeräten (K1 - K6), wobei wenigstens ein Bilderfassungsgerät (K5) den Cockpitbereich einschließlich des Tätigkeitsbereichs der Piloten abdeckt,
- einer bordeigenen Kommunikationseinheit (1) mit Eingängen für die Bilderfassungsgeräte (K1 - K6) sowie einer Speichereinheit (3) zur vorzugsweise komprimierten Abspeicherung der von den Bilderfassungsgeräten (K1 - K6) gelieferten Bilddaten,
- einer mit der bordeigenen Kommunikationseinheit in Datenaustausch stehenden stationären Kommunikationseinheit (6) mit daran angeschlossenen Einrichtungen (10, 11, 12, 13) zur Bilddatenverarbeitung und -wiedergabe,
- Sende- und Empfangseinrichtungen zur Übertragung zumindest der Bilddaten von der bordeigenen an die stationäre Kommunikationseinheit und Steuerdaten von der stationären an die bordeigene Kommunikationseinheit,
**dadurch gekennzeichnet, daß**
die stationäre Kommunikationseinheit (6) mit Mitteln zur Isolierung der Bilddaten und gegebenenfalls Steuerdaten aus der allgemeinen Funkleitstrahlinformation zwischen Flugzeug (7) und Bodenstation (8) versehen ist.

2. Überwachungseinrichtung nach Anspruch 1, **dadurch gekennzeichnet, daß** wenigstens ein Bilderfassungsgerät (K6) im Fracht- und Laderaum angeordnet ist.

3. Überwachungseinrichtung nach einem der Ansprüche 1 und 2, **gekennzeichnet durch** ein mobiles, an verschiedenen Stellen innerhalb des Flugzeuges einsetzbares Bilderfassungsgerät mit Anschluß an die bordeigene Kommunikationseinheit (1).

4. Überwachungseinrichtung nach Anspruch 1, **dadurch gekennzeichnet, daß** im Innenraum des Flugzeuges mindestens eine an die bordeigene Kommunikationseinheit (1) angeschlossene Eingabevorrichtung für einen Code, vorzugsweise für einen Zahlencode, angeordnet ist, daß die bordeigene Kommunikationseinheit (1) mit einem Vergleichsoperator zum Vergleich des eingegebenen Codes mit einem gespeicherten Nötigungscode versehen ist, und daß die bordeigene Kommunikationseinheit (1) mit einer im Falle einer Übereinstimmung zwischen eingegebenem Code und gespeichertem Nötigungscode ausgelösten Steuerung versehen ist, die selbsttätig eine Übertragung von Signalen und vorzugsweise Bilddaten an die stationäre Kommunikationseinheit (6) auslöst.

5. Überwachungseinrichtung nach Anspruch 4, **dadurch gekennzeichnet, daß** die Eingabevorrichtung der nur durch vorherige korrekte Codeeingabe zu öffnenden Tür zum Cockpit des Flugzeuges zugeordnet ist.

6. Überwachungseinrichtung nach einem der vorangehenden Ansprüche, **gekennzeichnet durch** einen an die bordeigene Kommunikationseinheit (1) angeschlossenen, vorzugsweise im Cockpit angeordneten Monitor (4) zur wahlweisen Wiedergabe der von den Bilderfassungsgeräten (K1 bis K6) gelieferten Bilddaten.

7. Überwachungseinrichtung nach Anspruch 6, **dadurch gekennzeichnet, daß** der im Cockpit angeordnete Monitor (4) mit einer automatischen Dunkelschaltung versehen ist.

8. Überwachungseinrichtung nach einem der vorhergehenden Ansprüche, **gekennzeichnet durch** ein Verfahren zur Bilddaten-Kompression mit einer Kompressionsrate größer als 1:100.

9. Überwachungseinrichtung nach einem der vorhergehenden Ansprüche, **dadurch gekennzeichnet, daß** die stationäre Kommunikationseinheit (6) mit Mitteln zur Erzeugung von Steuerdaten für den Betrieb der bordeigenen Kommunikationseinheit (1) versehen ist.

10. Überwachungseinrichtung nach Anspruch 9, **dadurch gekennzeichnet, daß** zur Steuerung der bordeigenen Kommunikationseinheit (1) und zur Bilddatenverarbeitung und -wiedergabe ein Personal Computer (10) mit Monitor (11) an die stationäre Kommunikationseinheit (6) angeschlossen ist.

11. Überwachungseinrichtung nach einem der vorangehenden Ansprüche, **dadurch gekennzeichnet, daß** als Sende- und Empfangseinrichtung des Flugzeugs (7) die vorhandene Bordantenne dient.

12. Überwachungseinrichtung nach einem der vorangehenden Ansprüche, **dadurch gekennzeichnet, daß** als stationäre Sende- und Empfangseinrichtung die land- oder satellitengestützten Einrichtungen der weltweit verbreiteten Bodenstation (8) dienen.

13. Überwachungseinrichtung nach einem der vorangehenden Ansprüche, **dadurch gekennzeichnet, daß** die Speichereinheit (3) mit einer spannungsversorgungs unabhängigen Datensicherung versehen ist, wie zum Beispiel einer magnetischen Festplatte, und daß sich die bordeigene Kommunikationseinheit (1) einschließlich der Speichereinheit (3) in einem geschlossenen Gehäuse (2) befindet, welches wasserdicht und stoßgesichert ist.

14. Überwachungseinrichtung nach einem der vorhergehenden Ansprüche, **gekennzeichnet durch** mindestens einen im Innenraum des Flugzeuges angeordneten Alarmtaster (T1 - T3) sowie eine Steuerung in der bordeigenen Kommunikationseinheit (1), die **durch** Betätigung des Alarmtasters (T1 - T3) selbsttätig eine Übertragung von Signalen und vorzugsweise Bilddaten an die stationäre Kommunikationseinheit (6) auslöst.

## Claims

1. Monitoring device for internal areas in aircraft, in particular passenger aircraft, having
- video detection appliances (K1 - K6) installed on board the aircraft, with at least one video detection appliance (K5) covering the cockpit area including the activity area of the pilots,
- an on-board communications unit (1) with inputs for the video detection appliances (K1 - K6) and with a memory unit (3) for preferably compressed storage of the video data supplied from the video detection appliances (K1 - K6),
- a stationary communications unit (6), which interchanges data with the on-board communications unit, and with devices (10, 11, 12, 13) connected to it for video data processing and replay,
- transmitting and receiving devices for transmitting at least the video data from the on-board communications unit to the stationary communications unit and control data from the stationary communications unit to the on-board communications unit,
**characterized in that**
the stationary communications unit (6) is provided with means for isolation of the video data and, possibly, control data from the general radio guidance-beam information between the aircraft (7) and the ground station (8).

2. Monitoring device according to Claim 1, **characterized in that** at least one video detection appliance (K6) is arranged in the freight and cargo area.

3. Monitoring device according to one of Claims 1 and 2, **characterized by** a mobile video detection appliance, which can be used at different points within the aircraft and has a connection to the on-board communications unit (1).

4. Monitoring device according to Claim 1, **characterized in that** at least one input apparatus for a code, preferably a numerical code, is arranged in the internal area of the aircraft and is connected to the on-board communications unit (1), **in that** the on-board communications unit (1) is provided with a comparison operator for comparing the entered code with a stored coercion code, and **in that** the on-board communications unit (1) is provided with a controller which is initiated if there is a match between the entered code and the stored coercion code and automatically initiates the transmission of signals, and preferably video data, to the stationary communications unit (6).

5. Monitoring device according to Claim 4, **characterized in that** the input apparatus is associated with the door, which can be opened only by previously entering the correct code, to the cockpit of the aircraft.

6. Monitoring device according to one of the preceding claims, **characterized by** a monitor (4), which is connected to the on-board communications unit (1) and is preferably arranged in the cockpit, for selective replay of the video data supplied from the video detection appliances (K1 to K6).

7. Monitoring device according to Claim 6, **characterized in that** the monitor (4) which is arranged in the cockpit is provided with automatic blanking.

8. Monitoring device according to one of the preceding claims, **characterized by** a method for video data compression, with a compression rate of more than 1:100.

9. Monitoring device according to one of the preceding claims, **characterized in that** the stationary communications unit (6) is provided with means for producing control data for operation of the on-board communications unit (1).

10. Monitoring device according to Claim 9, **characterized in that** a personal computer (10) with a monitor (11) is connected to the stationary communications unit (6), in order to control the on-board communications unit (1) and for video data processing and replay.

11. Monitoring device according to one of the preceding claims, **characterized in that** the existing on-board antenna is used as the transmitting and receiving device for the aircraft (7).

12. Monitoring device according to one of the preceding claims, **characterized in that** the land-based or satellite-based devices of the ground station (8), which are used throughout the world, are used as the stationary transmitting and receiving device.

13. Monitoring device according to one of the preceding claims, **characterized in that** the memory unit (3) is provided with data protection which is independent of any power supply, such as a magnetic hard disk, and **in that** the on-board communications unit (1), including the memory unit (3), is located in a closed housing (2), which is watertight and impact resistant.

14. Monitoring device according to one of the preceding claims, **characterized by** at least one alarm button (T1 - T3), which is arranged in the internal area of the aircraft, and by a controller in the on-board communications unit (1) which, as a result of operation of the alarm button (T1 - T3), automatically initiates transmission of signals and, preferably, video data to the stationary communications unit (6).

## Revendications

1. Dispositif de surveillance pour espaces intérieurs d'avions, notamment d'avions de transport de passagers, comportant
- des appareils de prise de vues (K1 à K6) installés à bord de l'avion, au moins un appareil de prise de vues (K5) couvrant la zone du cockpit, y compris la zone d'activité des pilotes,
- une unité de communication (1) embarquée à bord comportant des entrées pour les appareils de prise de vues (K1 à K6), ainsi qu'une unité de mémorisation (3) pour la mémorisation, de préférence à l'état compressé, des données d'image livrées par les appareils de prise de vues (K1 à K6),
- une unité de communication (6) stationnaire qui échange des données avec l'unité de communication embarquée à bord, à laquelle sont raccordés des dispositifs (10, 11, 12, 13) pour le traitement et la restitution de données d'image,
- des dispositifs d'émission et de réception destinés à la transmission des données d'image, au moins, de l'unité de communication embarquée à bord à l'unité de communication stationnaire, et de données de commande de l'unité de communication stationnaire à l'unité de communication embarquée à bord,
**caractérisé en ce que**
l'unité de communication (6) stationnaire comporte des moyens destinés à isoler les données d'image, et le cas échéant des données de commande, de l'information générale du faisceau radioélectrique entre l'avion (7) et la station au sol (8).

2. Dispositif de surveillance selon la revendication 1, **caractérisé en ce qu'**un appareil de prise de vues (K6) au moins est disposé dans l'espace de fret et de chargement.

3. Dispositif de surveillance selon l'une des revendications 1 et 2, **caractérisé par** un appareil de prise de vues mobile pouvant être utilisé à différents emplacements de l'avion, avec raccordement à l'unité de communication (1) embarquée à bord.

4. Dispositif de surveillance selon la revendication 1, **caractérisé en ce qu'**au moins un dispositif d'entrée pour un code, de préférence un code numérique, relié à l'unité de communication (1) embarquée à bord est disposé dans l'espace intérieur de l'avion, **en ce que** l'unité de communication (1) embarquée à bord est munie d'un opérateur de comparaison destiné à comparer le code entré avec un code d'urgence mémorisé, et **en ce que** l'unité de communication (1) embarquée à bord comporte une commande qui, en cas de concordance entre le code entré et le code d'urgence mémorisé, déclenche automatiquement une transmission de signaux, et de préférence de données d'image, à l'unité de communication (6) stationnaire.

5. Dispositif de surveillance selon la revendication 4, **caractérisé en ce que** le dispositif d'entrée est affecté à la porte du cockpit de l'avion qui ne peut être ouverte que par une entrée préalable du code correct.

6. Dispositif de surveillance selon l'une quelconque des revendications précédentes, **caractérisé par** un écran de contrôle (4) relié à l'unité de communication (1) embarquée à bord, qui est de préférence disposé dans le cockpit, pour la restitution, au choix, des données d'image délivrées par les appareils de prise de vues (K1 à K6).

7. Dispositif de surveillance selon la revendication 6, **caractérisé en ce que** l'écran de contrôle (4) disposé dans le cockpit comporte un variateur de luminosité automatique.

8. Dispositif de surveillance selon l'une quelconque des revendications précédentes, **caractérisé par** un procédé de compression de données d'image d'un taux de compression supérieur à 1/100.

9. Dispositif de surveillance selon l'une quelconque des revendications précédentes, **caractérisé en ce que** l'unité de communication (6) stationnaire comporte des moyens destinés à engendrer des données de commande pour le fonctionnement de l'unité de contrôle (1) embarquée à bord.

10. Dispositif de surveillance selon la revendication 9, **caractérisé en ce qu'**un ordinateur personnel (10) avec un écran de contrôle (11) est raccordé à l'unité de communication (6) stationnaire pour la commande de l'unité de communication (1) embarquée à bord, et pour le traitement et la restitution de données d'image.

11. Dispositif de surveillance selon l'une quelconque des revendications précédentes, **caractérisé en ce que** l'antenne existant à bord sert d'unité d'émission et de réception de l'avion (7).

12. Dispositif de surveillance selon l'une quelconque des revendications précédentes, **caractérisé en ce que** les infrastructures au sol ou assistées par satellite des stations au sol (8) mondialement répandues servent de dispositif d'émission et de réception stationnaire.

13. Dispositif de surveillance selon l'une quelconque des revendications précédentes, **caractérisé en ce que** l'unité de mémorisation (3) comporte une sauvegarde de données qui est indépendante d'une alimentation en tension, telle que par exemple un disque dur magnétique, et **en ce que** l'unité de communication (1) embarquée à bord, y compris l'unité de mémorisation (3), est logée dans un boîtier (2) étanche à l'eau et résistant aux chocs.

14. Dispositif de surveillance selon l'une quelconque des revendications précédentes, **caractérisé par** au moins un bouton-poussoir d'alarme (T1 à T3) disposé dans l'espace intérieur de l'avion, ainsi que par une commande dans l'unité de communication (1) embarquée à bord qui, par l'actionnement du bouton-poussoir d'alarme (T1 à T3), déclenche automatiquement une transmission de signaux, et de préférence de données d'image, à l'unité de communication (6) stationnaire.
